**Europäisches Patentamt**

(19) **European Patent Office**

**Office européen des brevets**

(11) Veröffentlichungsnummer: **0 210 125**
**B1**

(12) **EUROPÄISCHE PATENTSCHRIFT**

(45) Veröffentlichungstag der Patentschrift:
07.12.88

(51) Int. Cl.⁴: **A 61 B 17/39**

(21) Anmeldenummer: **86730109.5**

(22) Anmeldetag: **15.07.86**

(54) **Bipolare Elektrokoagulations-Fasszange.**

(30) Priorität: **23.07.85 US 758165**

(43) Veröffentlichungstag der Anmeldung:
**28.01.87 Patentblatt 87/5**

(45) Bekanntmachung des Hinweises auf die Patenterteilung:
**07.12.88 Patentblatt 88/49**

(84) Benannte Vertragsstaaten:
**DE FR GB IT**

(56) Entgegenhaltungen:
**EP-A- 0 119 405**
**FR-A- 2 535 196**

(73) Patentinhaber: **Tischer, Alfred, Dr.,**
**Potsdamer-Strasse 105, D-1000 Berlin 30 (DE)**

(72) Erfinder: **Tischer, Alfred, Dr., Potsdamer-Strasse 105,**
**D-1000 Berlin 30 (DE)**

(74) Vertreter: **Lüke, Dierck-Wilm, Dipl.-Ing.,**
**Gelfertstrasse 56, D-1000 Berlin 33 (DE)**

## Beschreibung

Die Erfindung bezieht sich auf eine bipolare Elektrokoagulations-Fasszange gemäss dem Oberbegriff des Anspruches 1.

Eine bipolare Elektrokoagulations-Fasszange dieser Art zum elektro-chirurgischen Trennen von Adhäsionen (Verwachsungen) ist aus der US-PS 2 068 721 vorbekannt. Bei dieser besteht die Schneideeinrichtung aus einem elektrisch beheizbaren Draht. Dieser wird nach dem Koagulieren zweier im Abstand voneinander befindlicher Gewebeteile mittels der beiden schmalen, traumatischen Zangenschenkel durch deren Öffnungen hindurchgeführt, woraufhin der elektrisch beheizbare Schneidedraht, der eine gewisse Aufheiz- und Abkühlzeit benötigt, das Gewebe zerschneidet. Somit sind nur das Erfassen, Koagulieren und Zerschneiden des Gewebeteiles möglich. Eine Gewebeentnahme aus dem Körper ist mit dieser bekannten Fasszange nicht möglich. Insbesondere ist eine Verwendung für die Tubenligatur nicht möglich, da die Tuben stark blutdurchflossen sind und bereits beim Koagulieren mittels der schmalen, gezahnten und daher traumatischen Zangenschenkel beschädigt werden, so dass bereits beim Koagulieren ein Schneidevorgang erfolgt.

Es ist aus der US-PS 4 369 788 ein gattungsfremdes medizinisches Schneidinstrument zur Gewebeentnahme bekannt, welches einen festen Zangenschenkel und ein gegenüber diesem bewegbares, im Querschnitt U-förmiges Schneidemesser aufweist, zwischen dessen Schneiden eine Aufnahmemulde für herausgeschnittene Gewebeteile ausgebildet ist. Dieses medizinische Instrument weist jedoch keine Zangenschenkel zum Ergreifen und Koagulieren menschlicher oder tierischer Gewebeteile auf; mit diesem kann deshalb auch kein Gewebe koaguliert und anschliessend durchtrennt werden.

Der Erfindung liegt von daher die Aufgabe zugrunde, eine bipolare Elektrokoagulations-Fasszange der gattungsgemässen Art dahingehend auszubilden, dass sowohl ein Ergreifen, Koagulieren und Schneiden von Gewebeteilen als auch eine Gewebeentnahme möglich sind, wobei die Fasszange insbesondere zur Tubensterilisation mittels bipolarer Hochfrequenzwärme ausgebildet und das Gewebe der koagulierten Tuben zur Gewebeuntersuchung mit der Fasszange herausnehmbar sein soll.

Die Lösung dieser Aufgabe ergibt sich aus den kennzeichnenden Merkmalen des Anspruches 1. Mit der erfindungsgemässen bipolaren Elektrokoagulations-Fasszange ist es möglich, sowohl ein Gewebeteil, insbesondere eine Tube, an zwei im Abstand voneinander befindlichen Stellen zu koagulieren, d.h. elektrisch mittels bipolaren HF-Stromes zu verschweissen, das zwischen den beiden Koagulationsstellen befindliche Gewebe der Tube abzutrennen und das abgetrennte Gewebe schliesslich zu entnehmen und der histologischen Untersuchung zur Identifizierung zuzuführen. Bei Verwendung der erfindungsgemässen Fasszange zur Tubensterilisation ist es besonders vorteilhaft, dass nur ein besonders kurzer Teil der Tube koaguliert wird, so dass später erforderlichenfalls eine wiederherstellende Operation möglich ist.

Die Anwendung der erfindungsgemässen bipolaren Elektrokoagulations-Fasszange ist äusserst einfach und vorteilhaft. Nach üblicher medizinischer Vorbereitung, insbesondere einer Tubensterilisation, wird die Fasszange im geschlossenen Zustand an die Tube herangeführt. Anschliessend werden sowohl der bewegliche Zangenschenkel als auch das Schneidemesser geöffnet, wobei das Schneidemesser in der Öffnung des geöffneten beweglichen Zangenschenkels aufgenommen wird. Das geöffnete Schneidemesser dient als Anschlag für die nun erfasste Tube. Anschliessend wird der bewegliche Zangenschenkel wieder auf den festen Zangenschenkel gedrückt, wobei die Tube zwischen beiden Zangenschenkeln eingeklemmt wird, während das Schneidemesser in seiner geöffneten Stellung verbleibt und die Öffnung des beweglichen Zangenschenkels weiterhin durchdringt. Mittels der Hochfrequenz-Gleichspannung erfolgt die Koagulation der zwischen den beiden Zangenschenkeln eingeklemmten Tube. Nach Vollendung der Koagulation der beiden im Abstand voneinander befindlichen Tubenbereiche wird das Schneidemesser betätigt, welches aufgrund seiner U-form den zwischen den beiden Koagulationsbereichen liegenden Bereich der Tube herausschneidet und in die innerhalb der Öffnung des festen Zangenschenkels befindliche Aufnahme drückt. Die nunmehr geschlossene Fasszange wird aus dem Körper des Patienten herausgezogen. Anschliessend kann der in der Aufnahme befindliche Gewebeabschnitt der Tube einer histologischen Untersuchung zur Identifikation der Tubenligatur zugeführt werden.

Mit der erfindungsgemässen Fasszange wird somit mit ein und demselben medizinischen Instrument, also ohne jeden Instrumentenwechsel, innerhalb des koagulierten Bereiches, d.h. im blutleeren Bereich des Gewebes der Tube, ein Gewebeanteil herausgeschnitten. Dieser kann mit dem gleichen Instrument aufgefangen, gehalten und nach Entfernung des Instrumentes aus dem Körper des Patienten mühelos dem Instrument entnommen werden. Somit können mit einem einzigen medizinischen Instrument die Arbeitsgänge Fassen, bipolares Koagulieren, Schneiden und Entnahme eines Gewebeanteiles der Tube einzeln und unabhängig voneinander unmittelbar nacheinander ausgeführt werden.

Weitere vorteilhafte Ausgestaltungen der Erfindung ergeben sich aus den Unteransprüchen.

Die Erfindung ist nachfolgend anhand eines in den Zeichnungen dargestellten Ausführungsbeispieles einer bipolaren Elektrokoagulations-Fasszange näher erläutert. Es zeigen:

Fig. 1 eine Seitenansicht des die Handgriffe aufweisenden hinteren Endes der Fasszange,

Fig. 2 eine Seitenansicht des die Zangenschenkel aufnehmenden vorderen Endes der Fasszange in geöffneter Stellung und

Fig. 3 eine Draufsicht auf das vordere Ende der Fasszange in geöffneter Stellung.

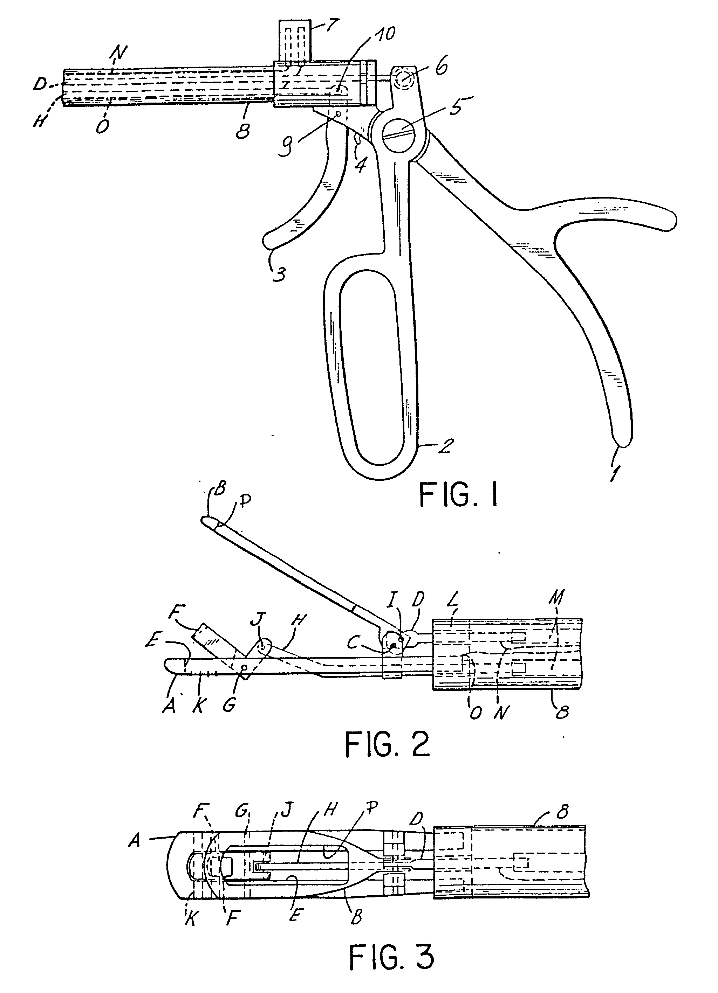

Die bipolare Elektrokoagulations-Fasszange zum Erfassen, Koagulieren mittels bipolarer Hochfrequenzwärme, Schneiden und zur Entnahme von menschlichem oder tierischem Gewebe, dient insbesondere zur Tubensterilisation. Die Fasszange weist an ihrem vorderen Ende die in den Figuren 2 und 3 dargestellte Funktionseinheit auf, die aus einem festen Zangenschenkel A, einem bewegbaren Zangenschenkel B und aus einer Schneideeinrichtung F gebildet ist, von denen der bewegbare Zangenschenkel B und die Schneideeinrichtung F gegenüber dem festen Zangenschenkel A und gegeneinander bewegbar sind. Der bewegbare Zangenschenkel B und die bewegbare Schneideeinrichtung F sind über Zug- und Schubstangen D bzw. H, die in einem Führungsrohr 8 gelagert sind, vom hinteren Ende, der Betätigungseinheit der Fasszange aus betätigbar.

Das in Fig. 1 dargestellte hintere Ende der Fasszange bildet die Betätigungseinheit. Diese besteht aus einem am Führungsrohr 8 befestigten Griff 1, einem an diesem und gegenüber diesem um ein Gelenk 5 bewegbaren zweiten Griff 2, der nach Art eines Scherengriffes ausgebildet ist, und aus einem dritten, hakenartigen Griff 3, der um ein Gelenk 9 am festen Griff 1 bewegbar ist. Der zweite bewegbare Griff 2 ist über ein Gelenk 6 mit der Zug- und Schubstange D verbunden, welche in der Funktionseinheit den bewegbaren Zangenschenkel B bewegt. Der dritte, hakenartige Griff 3 der Betätigungseinheit ist über ein Gelenk 10 mit der Zug- und Schubstange H verbunden, welche in der Funktionseinheit die Schneideeinrichtung F betätigt. Somit umfasst die Betätigungseinheit drei Griffe 1 bis 3, von denen die Griffe 2 und 3 am festen Griff 1 bewegbar angelenkt und gegenüber diesem schwenkbar sind. Eine Schliessbewegung der Griffe 1, 2, d.h. eine Bewegung des bewegbaren Griffes 2 auf den festen Griff 1 zu, schliesst die Zangenschenkel A, B und öffnet diese in entgegengesetzter Bewegungsrichtung. Entsprechend schliesst eine Bewegung des bewegbaren Griffes 3 in Richtung auf den festen Griff 1 die Schneideeinrichtung F und öffnet diese in umgekehrter Richtung. Der Schwenkweg des bewegbaren Griffes kann kann mittels eines Einstellelementes 4, das am festen Griff 1 angeordnet ist, eingestellt werden.

In der Betätigungseinheit der Fasszange ist benachbart zu den Griffen 1 bis 3 auf dem Führungsrohr 8 eine Steckbuchse 7 angeordnet, welche gegenüber dem Führungsrohr 8 isoliert ist und welche zur Verbindung mit einer Hochfrequenz-Spannungsquelle und somit zur Zufuhr von hochfrequenter Gleichspannung dient. Innerhalb des Führungsrohres 8 sind elektrische Leiter N und O geführt, welche die beiden Kontakte der Steckbuchse 7 mit den Zangenschenkeln A und B elektrisch verbinden. Das Führungsrohr 8 ist auf seiner Innenseite mit einer Isolationsschicht ausgekleidet.

Die am vorderen Ende der Fasszange ausgebildete, in den Figuren 2 und 3 näher dargestellte Funktionseinheit wird nun näher beschrieben. Diese dient zur Ausführung folgender Funktionen: Ergreifen einer Tube, Koagulieren der Tube an zwei benachbarten, im Abstand der Breite der Zangenschenkel A, B befindlichen Stellen, Herausschneiden des zwischen den Koagulationsbereichen befindlichen Tubenstückes und Entnehmen der herausgeschnittenen Tubenstücke zur histologischen Untersuchung zwecks Identifizierung der Güte der Tubenligatur.

Die Funktionseinheit umfasst die beiden Zangenschenkel A, B und die Schneideeinrichtung F. Der feste Zangenschenkel A dient als Anschlag für den bewegbaren Zangenschenkel B. Dieser ist mittels eines Gelenkes C am festen Zangenschenkel A gelagert. Die beiden Zangenschenkel A und B dienen als Elektroden für den Hochfrequenzstrom zur Elektrokoagulation. Der bewegbare Zangenschenkel B ist zur elektrischen Isolation gegenüber dem Zangenschenkel A im Gelenk C mittels einer Kunststoffhülse gelagert. Die Zug- und Schubstange D für den bewegbaren Zangenschenkel B ist im Gelenk I mit diesem verbunden. Folglich kann der bewegbare Zangenschenkel B unter Wirkung der Zug- und Schubstange D nach Betätigung des bewegbaren Griffes 2 um einen Öffnungswinkel von bis zu etwa 60° gegenüber dem festen Zangenschenkel A geschwenkt werden, wodurch ein sicheres Ergreifen der Tube ermöglicht wird.

Sowohl der feste Zangenschenkel A als auch der bewegbare Zangenschenkel B sind mit Öffnungen E bzw. P in Form von Längsschlitzen versehen, welche insbesondere in Fig. 3 dargestellt sind. Innerhalb der Öffnung E des festen Zangenschenkels A ist die Schneideeinrichtung F drehbar um ein Gelenk G angeordnet, welche L-förmig ausgebildet ist und über ein Gelenk I mit der Zug- und Schubstange H verbunden ist, welche wiederum in der Bedienungseinheit mit dem bewegbaren Hebel 3 gelenkig verbunden ist. Die Öffnungsbewegung der Schneideeinrichtung F gegenüber dem festen Zangenschenkel A ist ebenfalls um einen Winkel von bis zu etwa 60° möglich. In der geschlossenen Lage ist die Schneideeinrichtung F vollständig innerhalb der Öffnung E des festen Zangenschenkels A aufgenommen. Die Schneideeinrichtung F ist als ein gebogenes, U-förmiges Schneidemesser ausgebildet, dessen seitliche Schneidekanten parallel zu den Längsseitenkanten der Öffnung E des festen Zangenschenkels A liegen.

Der bewegbare Zangenschenkel B und der feste Zangenschenkel A sind über das Gelenk C derart angeordnet, dass diese sich gleichförmig entlang der gesamten Länge ihres Greifbereiches im wesentlichen parallel zueinander nähern, wozu das Gelenk C in einem geringen Abstand oberhalb des festen Zangenschenkels A angeordnet ist. Somit wird die Tube durch eine Bewegung des bewegbaren Zangenschenkels B gleichmässig auf den festen Zangenschenkel A gepresst, so dass der gesamte ergriffene Bereich der Tube gleichförmig koaguliert werden kann.

Wie es in Fig. 2 dargestellt ist, ist unterhalb der Öffnung E des festen Zangenschenkels A eine Aufnahme in Form eines Drahtkorbes K ausgebildet, um die abgeschnittenen Tubenteile aufzufangen. Das U-förmig gebogene Schneidemesser der Schneideeinrichtung F ist derart ausgebildet, dass der herausgeschnittene Tubenbereich des Schneidevorganges in den Drahtkorb K hineingedrückt wird. Der Drahtkorb K kann mehrere Tubenabschnitte aufnehmen, so dass eine vollständige Sterilisation ohne Wechsel der Fasszange ausgeführt werden kann, d.h. es können beide Tuben nacheinander operiert werden.

Der feste Zangenschenkel A ist starr mit dem Führungsrohr 8 über eine Isolierhülse L verbunden. Die Isolierhülse L dient gleichzeitig zur Führung der Zug-und Schubstangen D und H, welche die Isolierhülse L innerhalb von Durchgangsbohrungen durchdringen. Innerhalb des Führungsrohres 8 ist ein Längsabschnitt jeder der Zug- und Schubstangen D, H als Isolierstange M ausgebildet.

Eine laparoskopische Tubenligatur wird mit der vorstehend beschriebenen Elektrokoagulations-Fasszange wie folgt ausgeführt.

Nach der Ausbildung eines Pneumoperitoneums und der Einführung eines optischen Instrumentes in den zu operierenden menschlichen oder tierischen Körper wird die Fasszange mit ihrer Funktionseinheit in den Körper-Hohlraum mittels eines zweiten Einstiches mit einem konventionellen 10 cm Trokar eingeführt. Die Steckbuchse 7 der Fasszange ist mit einer üblichen bipolaren Hochfrequenz-Spannungsquelle verbunden.

Nach dem Lokalisieren der Tube werden der bewegbare Zangenschenkel B und das Schneidemesser F gleichzeitig geöffnet, wobei das geöffnete und gemäss Fig. 2 schräg gestellte Schneidemesser F als Anschlag für die Tube dient. Anschliessend wird die Tube durch Schliessen des bewegbaren Zangenschenkels B gegen den festen Zangenschenkel A gepresst, wobei das Schneidemesser F durch die Öffnung P des bewegbaren Zangenschenkels B hindurchragt. Nun wird der Hochfrequenzstrom über die beiden Zangenschenkel A und B zur bipolaren Koagulation der Tube zugeführt, so dass die Tube zwischen den Zangenschenkeln A und B an zwei voneinander im Abstand befindlichen Stellen koaguliert wird. Der Abstand der beiden Koagulationsbereiche ist durch die Öffnungsweite der beiden Öffnungen P und E der Zangenschenkel B bzw. A vorgegeben. Nach der Koagulation der Tube, d.h. dem elektrischen Verschweissen mittels bipolaren HF-Stromes wird bei weiterhin geschlossen gehaltenen Zangenschenkeln A, B das Schneidemesser F betätigt. Der Tubenabschnitt zwischen den beiden koagulierten Bereichen wird herausgeschnitten und mittels des im Querschnitt U-förmigen Schneidemessers F in den Drahtkorb K des festen Zangenschenkels A hineingepresst. Somit ist der Operationsabschnitt zur Tubensterilisation einer Tube abgeschlossen. Die Gewebsprobe dieser Tube befindet sich im Drahtkorb K.

Zur Vervollständigung der Sterilisation wird der gleiche Vorgang auch an der zweiten Tube durchgeführt, ohne dass die Fasszange gewechselt werden muss. Sobald die Fasszange aus der Körperhöhle entfernt worden ist, werden die beiden Gewebsproben, die aufgrund der aufeinanderfolgenden Operationen übereinander im Drahtkorb K liegen, entnommen und einer histologischen Untersuchung zugeführt. Dies ist besonders aus forensischen Gründen erforderlich.

## Patentansprüche

1. Bipolare Elektrokoagulations-Fasszange zum Ergreifen, Koagulieren mittels bipolarer Hochfrequenzwärme und Schneiden von menschlichem oder tierischem Gewebe, insbesondere für die Tubenligatur, aus einem festen Zangenschenkel und aus einem gegenüber diesem elektrisch isolierten, bewegbaren Zangenschenkel, wobei in beiden Zangenschenkeln gegenüberliegende Öffnungen ausgebildet und die Zangenschenkel mit Anschlüssen an die Pole einer HF-Gleichspannungsquelle versehen sind, und aus einer relativ zu den Zangenschenkeln gelenkig bewegbar gelagerten Schneideeinrichtung zum Schneiden der Gewebeteile zwischen den beiden durch die Öffnungen der Zangenschenkel begrenzten Koagulationsbereichen, dadurch gekennzeichnet, dass die Schneideeinrichtung als U-förmig gebogenes Schneidemesser (F) ausgebildet und drehbar mittels eines Gelenks (G) innerhalb der Öffnung (E) des festen Zangenschenkels (A) gelagert und in ihrer geschlossenen Lage in die Öffnung (E) des festen Zangenschenkels (A) versenkbar ist und dass innerhalb der Öffnung (E) des festen Zangenschenkels (A) eine Aufnahme (K) für mittels des U-förmigen Schneidemessers (F) herausgeschnittene und in die Aufnahme (K) hineingedrückte Gewebeabschnitte vorgesehen ist.

2. Bipolare Elektrokoagulations-Fasszange nach Anspruch 1, dadurch gekennzeichnet, dass die beiden Zangenschenkel (A, B) mit breiter Auflagefläche und atraumatisch ausgebildet sind.

3. Bipolare Elektrokoagulations-Fasszange nach Anspruch 1 oder 2, dadurch gekennzeichnet, dass die Aufnahme (K) als die im festen Zangenschenkel (A) ausgebildete Öffnung (E) nach aussen abschliessender Drahtkorb ausgebildet ist.

4. Bipolare Elektrokoagulations-Fasszange nach einem der Ansprüche 1 bis 3, dadurch gekennzeichnet, dass die Aufnahmeöffnung (E) des festen Zangenschenkels (A) zum Schneiden des Gewebes entsprechend der U-Form des Schneidemessers (F) U-förmig ausgebildet ist.

5. Bipolare Elektrokoagulations-Fasszange nach einem der Ansprüche 1 bis 4, dadurch gekennzeichnet, dass das U-förmig gebogene Schneidemesser (F) innen konisch ausgeformt ist.

## Claims

1. A bipolar electro-coagulating barrel forceps for gripping, coagulating by bipolar high frequency heating and cutting of human or animal tissues, especially for ligature of tubes, comprising of a fixed forceps arm and a movable forceps arm electrically insulated therefrom, wherein opposing openings are made in both forceps arms and the forceps arms are provided with connections to the poles of a high frequency direct current source, and a cutting device, fastened pivotably movable relative to the forceps arms, to cut the portion of tissue between the coagulation regions limited by the openings in the forceps arms, characterized in that the cutting device is formed as a U-shaped curved cutting knife (F) and is povitably fixed by means of a joint (G) within the opening (E) of the fixed forceps arm (A) and in the closed position is retractable into the opening (E) of the fixed forceps arm (A) and that within the opening (E) of the fixed forceps arm (A) a receiver (K) is provided for the section of tissue excised by means of the U-shaped cutting knife (F) and pressed into the receiver (K).

2. A bipolar electro-coagulating barrel forceps ac- according to Claim 1, characterized in that both forceps arms (A, B) are made with broad bearing-surface areas and are non-traumatizing.

3. A bipolar electro-coagulating barrel forceps according to Claim 1 or 2, characterized in that the receiver (K) is formed as the externally closed wire basket of the opening (E) made in the fixed forceps arm (A).

4. A bipolar electro-coagulating barrel forceps according to any one of Claims 1 to 3, characterized in that the receiving opening (E) of the fixed forceps arm (A) for cutting tissue is made U-shaped corresponding to the U-shape of the cutting knife (F).

5. A bipolar electro-coagulating barrel forceps according to any one of Claims 1 to 4, characterized in that the U-shaped curved cutting knife (F) is internally conical.

**Revendications**

1. Pince bipolaire d'électro-coagulation pour la saisie, la coagulation par échauffement à haute fréquence bipolaire et découpe de tissus humaines ou animaux, en particulier pour la ligature de vaisseaux, par une branche en pince fixe et une branche de pince mobile isolée électriquement de la première, où des ouvertures se faisant face sont pratiquées sur les deux branches, et celles-ci sont munies de bornes reliées aux pôles d'une source de tension continue à haute fréquence, et d'un système de coupe supporté de façon mobile articulé par rapport aux branches de pince pour couper les portions de tissu entre les deux zones de coagulation limitées par les ouvertures des branches de pince, caractérisée en ce que la lame de coupe (F) est recourbée en forme de U et est supportée de façon rotative au moyen d'une articulation G à l'intérieur de l'ouverture (E) de la branche de pince fixe (A) et peut s'enfoncer en position fermée dans l'ouverture (E) de la branche fixe (A), et en ce qu'à l'intérieur de l'ouverture (E) de la branche fixe (A) est prévue une cavité (K) pour la section de tissu coupée par la lame de coupe (F) en forme de U et poussée dans la cavité (K).

2. Pince bipolaire d'électro-coagulation selon la revendication 1, caractérisée en ce que les deux branches de pince (A, B) sont constituées de surfaces de portée plus large et non traumatisantes.

3. Pince bipolaire d'électro-coagulation selon la revendication 1 ou la revendication 2, caractérisée en ce que la cavité (K) est formée d'un panier de fil se fermant vers l'extérieur dans l'ouverture (E) de la branche fixe (A).

4. Pince bipolaire d'électro-coagulation selon l'une des revendications 1 à 3, caractérisée en ce que l'ouverture de réception (E) de la branche fixe (A) pour la coupe du tissu est d'une forme correspondant à la forme en U de la lame de coupe (F).

5. Pince bipolaire d'électro-coagulation selon l'une des revendications 1 à 4, caractérisée en ce que la lame de coupe (F) recourbée en forme de U présente une conformation interne conique.

FIG. I

FIG. 2

FIG. 3